# EUROPEAN PATENT SPECIFICATION

(11) **EP 1 203 080 B1**
(45) Date of publication and mention of the grant of the patent: **17.11.2004**
(21) Application number: 00953272.2
(22) Date of filing: 24.07.2000
(51) Int. Cl.: C12N 15/10, C12M 1/26, B01L 3/00, G01N 1/28

(54) **ISOLATION OF BIOMOLECULES**
ISOLIERUNG VON BIOMOLEKÜLEN
ISOLEMENT DE BIOMOLECULES

(30) Priority: 23.07.1999 GB 9917299; 12.02.2000 GB 0003282
(43) Date of publication of application: 08.05.2002
(73) Proprietor: TEPNEL MEDICAL LIMITED, Altrincham WA14 5UA (GB)
(72) Inventor: BROWN, Allan, Didsbury Manchester M20 2TA (GB); LOCK, Stephen Owen, Hartford Northwich CW8 4AX (GB); DODSWORTH, Steven James, Moss Clwyd LL11 6DR (GB); HUDSON, Ian Tepnel Life Sciences, Didsbury Manchester M20 2RY (GB)
(74) Representative: Atkinson, Peter Birch
(86) International application number: PCT/GB2000/002798
(87) International publication number: WO 2001/007451

(56) References cited:
- WO-A-95/02049
- WO-A-96/26782
- WO-A-97/26540
- WO-A-98/42874
- US-A- 3 985 032
- US-A- 5 496 523
- US-A- 5 645 723

## Description

The present invention relates to a purification method and apparatus for use in obtaining samples of biomolecules. The invention relates more particularly, but not exclusively, to such method and apparatus which may be used for obtaining samples of nucleic acids or proteins.

Numerous methods are known for obtaining biomolecules, for example nucleic acids and proteins, from biological material such as viruses, bacterial and eukaryotic cells, cell aggregates and tissue or body fluids. Typically the biomolecule to be obtained is a soluble molecule and is "released" from the biological material by a lysis procedure (e.g. alkaline lysis) resulting in a suspension comprised of a solution of the target biomolecule also containing soluble proteins, carbohydrates, fats, amino acids and other metabolites from the disrupted cells.

In many methods known in the art for effecting the purification of a desired biomolecule, those molecules which would otherwise contaminate the desired product are insoluble, or are rendered so by a chemical process. The insoluble material is then removed by methods known in the art (e.g. centrifugation and aspiration of the supernatant) to achieve a degree of purification of the soluble material. These insoluble materials may include, for example, whole cells, or fragments thereof, flocculated proteins and unwanted nucleic acid material (e.g. chromosomal DNA contamination of a plasmid DNA preparation).

After separation of the insoluble material, from the solution, the latter is applied to a solid phase binding matrix under conditions (e.g. in the presence of a chaotropic salt) such that the matrix binds the biomolecule of interest. Subsequently the solution is removed from the matrix (leaving the biomolecule bound thereto), the matrix washed to remove non-bound material, and the biomolecule eluted.

The separation of the insoluble material from the solution is generally effected by centrifugation of the suspension followed by careful pipetting. However the use of centrifugation is a disadvantage in that it does not easily allow the procedure for obtaining the biomolecule to be fully automated. Furthermore, the separation of soluble from insoluble material by pipette is awkward to perform and must be carried out accurately to prevent unwanted insoluble material from being added to the solid phase matrix and causing contamination, see also the patent applications US 3 985 032 and WO 97/26540.

One purification method which seeks to overcome some of the problems associated with conventional techniques is disclosed in WO-A-95/02049. The apparatus disclosed involves a pneumatic delivery system which is used to add, mix and remove reagents from a flow-through vessel in order to separate a target biomolecule from cells. The vessel has two chambers between which is a porous membrane or filter. The membrane functions to retain cells and cellular debris and insoluble material in the upper chamber whilst soluble material is filtered through and further purified by binding to a solid phase matrix present in the lower chamber.

There are however disadvantages associated with the technique disclosed in WO-A-95/02049. In particular. if it is desired to treat the solid phase matrix (with biomolecule bound thereto) with additional solutions (e.g. wash or elution solutions) it is necessary to pass those solutions into the lower chamber either via the upper chamber and filter (which retains insoluble material) or provide additional inlets in the lower chamber. In the former case, there is the risk of contamination. In the latter case, the provision of additional ports in the lower chambers makes the apparatus difficult to manufacture.

It is an object of the present invention to obviate or mitigate the aforementioned disadvantages.

According to a first aspect of the present invention there is provided a method of obtaining a sample containing the biomolecule from a suspension comprising a solution of the biomolecule and insoluble material, the method comprising the steps of:
(a) providing a biomolecule purification assembly comprised of a vessel having a liquid inlet and of a filter unit removably located on the liquid inlet;
(b) effecting a filtration of the suspension through the filter unit so as to cause the solution to enter the vessel through the liquid inlet;
(c) removing the filter unit from the liquid inlet;
(d) immobilising the biomolecule on a solid phase support; and
(e) subjecting the molecule to at least one of the steps of washing on the support and elution from the support to obtain a purified sample of the biomolecule.

According to a second aspect of the present invention there is provided apparatus for obtaining a sample of a biomolecule from a suspension comprising a solution of the biomolecule and insoluble material, the apparatus comprising
(i) a biomolecule purification assembly comprised of a vessel having a liquid inlet and of a filter unit removably located on the liquid inlet of the vessel;
(ii) means for causing the solution present in the suspension to pass through the filter unit and the liquid inlet into the vessel; and
(iii) means for removing the filter unit from the vessel.

According to a third aspect of the present invention there is provided a biomolecule purification assembly comprising a vessel having a liquid inlet and a filter unit removably located on the liquid inlet wherein the vessel is in the form of a column having a first bore section and a second bore section of reduced diameter as compared to the first section, and the filter unit is comprised of a sleeve which houses the filter and which is removably located on the end of the vessel remote from the first bore section.

In accordance with the invention therefore separation of insoluble material from the suspension is effected through a filter unit which is removably located in the liquid inlet of a vessel and a filtration is effected causing the solution to enter the vessel. Any insoluble impurities in the suspension are retained on the filter unit. Subsequently, the filter is removed from the inlet and is preferably discarded rather than being reused. Thus each filter need only be used once avoiding problems of contamination.

The method of the invention negates the requirement for the suspension to be centrifuged to separate soluble and insoluble materials in the suspension. The method further negates the requirement for accurate separation of the soluble and insoluble phases by use of a pipette. Furthermore, the method of the invention is relatively simple to perform and is eminently suited to automation as will be appreciated from the description given below.

It is preferred that, in step (e) of the method, the step of washing or elution is effected on solid phase support contained within the vessel. Preferably both of the steps of washing and elution are effected, and the biomolecule of interest is eluted through said inlet of the vessel.

It is particularly preferred that the solid phase support material is a particulate or bead-like material which is introduced into the vessel (preferably through said inlet) after the step of removing the filter unit therefrom.

The vessel of the biomolecule purification assembly is preferably a flow through vessel, most preferably an open-ended column, e.g. having a volume sufficient to hold 0.25 to 1.5ml of sample, disposed vertically so that its lower end provides the aforementioned liquid inlet and the upper end may be used for the introduction of additional reagents into the column as required.

The vessel may comprise upper and lower bore sections whereof the diameter of the upper section is greater than that of the lower section, the two sections being connected by a intermediate, tapering bore section, the purpose of the reduced section lower bore will be described below.

The filter unit may be of any material capable of tolerating the reagents used and will comprise a filter element having a pore size which is capable of preventing passage of the insoluble material of the composition therethrough but which is not so small that the flow rate through the filter becomes unacceptably low. Typically the pore size of the element will be in the range 0.2 to 50 microns.

The filter unit may, for example, comprise a sleeve or the like for location over the liquid inlet of the vessel and a filter located in the sleeve. The filter unit may for example be a push fit or a loose snap fit over the liquid inlet.

Preferably the filter incorporates a depression locating in close proximity to the liquid inlet of the vessel. This allows filtrate to come into close proximity with the inlet and enhance the rate of filtrate uptake into the vessel.

A preferred embodiment of filter unit (which also provides, in its own right, a fourth aspect of the invention) comprises
(a) an elongate sleeve having one end (the "suspension inlet end") through which a suspension to be filtered enters the sleeve and an opposite end for location over a column of a biomolecule purification assembly, said sleeve having a first body portion and a second body portion which internally tapers from the first body portion to said suspension inlet end, and
(b) a filter having a head portion locating in. and occupying the cross-section of, the first body portion, and a frustoconical or conical body portion extending into said second body portion of the sleeve and tapering therein at a larger angle than the internal taper of the sleeve.

The construction of filter unit as defined in the previous paragraph has a number of advantages as detailed below.

Firstly, there is clearance between the outer surface of the conical or frustoconical portion of the filter and the inner wall of the tapering portion of the sleeve. This ensures that there is a relatively large area of the filter exposed to the suspension to be filtered so that the filter unit has as high a capacity for filtration as possible given the overall dimensions of the filtration unit.

Secondly, the "dead space" within the filter unit (i.e. the space between the outer surface of the conical or frustoconical portion of the filter and the tapering section of the sleeve) may be relatively low whilst still allowing the advantage of the previous paragraph. The minimum dead space ensures that there is a minimum liquid remaining (in the dead space) when all of the suspension to be filtered has been drawn up into the filter unit and air is about to enter the column.

Thirdly, the elongate nature of the filter unit ensures that it's lowermost, suspension inlet end may be juxtaposed to the base of a microtitre well or the like.

Overall, therefore. the filter unit allows maximum amount of suspension to be filtered, with high filtration efficiency, before air enters the unit.

Preferably the first body portion of the sleeve is of circular internal cross-section and the head of the filter is also cylindrical so as to be a close fit therein.

The filtration step of the method of the invention may be effected in a number of ways. Thus for example, a reduced pressure may be applied to the interior of the vessel causing solution from the suspension to be drawn through the filter unit into the vessel.

As an alternative to the use of reduced pressure, the suspension may be provided in an open-topped container within which the filter unit is a close sliding fit such that by moving the filter unit within the container towards the base thereof solution is caused to be forced through the filter unit into the vessel for binding of the biomolecule as described above.

Subsequent to the filtration operation, the filter unit is removed from the vessel.

It is particularly preferred in accordance with the invention that the suspension to be filtered is contained in a well and that once the filtration operation is complete the filter unit is automatically discarded into that well. This may be achieved in a number of ways. Thus, for example, the biomolecule purification assembly may be lowered towards the well to effect filtration and move upwardly after filtration is completed and the apparatus may incorporate a stripping arrangement which acts on the filter unit as the biomolecule purification assembly is moved upwardly causing the filter unit to be discharged into the well. Alternatively, the filter unit and microtitre well have inter-engagable formations whereby as the biomolecule purification assembly is lowered towards said well the formations come into an engaging relationship requiring a greater force to release the engagement than is required for removing the filter unit from the vessel. Thus, as the vessel is moved upwardly away from the well, the engagement is maintained and the filter unit is removed from the vessel and retained in the well.

Subsequent to the removal of the filter unit, the biomolecule is immobilised in a solid phase support (i.e. step (d) of the method), preferably in the form of particles or beads having a cross-sectional size (e.g. diameter) of 0.05 to 250 microns (e.g. 0.1 to 250 microns). Preferably the particles do not fill the entire space between the retaining means so that the particles may be "fluidised" within the vessel.

The immobilisation of the biomolecule onto the solid phase support is preferably effected in the presence of a binding agent composition. In the case that the biomolecule to be immobilised is a nucleic acid, the binding agent composition may for example comprise polyethyleneglycol together with sodium and potassium cations and chloride and acetate anions. Alternatively the binding agent formulation may comprise a chaotropic salt or other agents capable of effecting absorption of the biomolecule onto the support. By chaotropic salt it is meant any substance capable of altering the secondary, tertiary and/or quaternary structure of a protein or nucleic acid molecule, but leaving at least the primary structure intact. Examples of chaotropic salts which may be utilised to allow binding of nucleic acid or proteins to the solid phase binding matrix are guanadinium salt, sodium iodide, potassium iodide, sodium (iso)thiocyanate, urea or combinations thereof. Preferred chaotropic salts for use in the present invention include guanidinium hydrochloride and guanidinium (iso)thiocyanate. For the purposes of effecting step (d) of the method (i.e. immobilising the biomolecule on the solid phase support), the filtrate in the vessel may contain the chaotropic salt or other immobilising agent(s) and the filtrate is discharged onto the support material prior to the resultant mixture being taken back into the vessel. Alternatively, the filtrate may be discharged into a mixture of the support and the binding agent composition and the resultant mixture is then taken back into the vessel.

Subsequently, the mixture of the filtrate, binding agent composition and the beads may be drawn back up into the vessel and, if desired. may be subjected to at least one cycle of discharge from. and uptake back into. the vessel to improve mixing.

It is preferred that the support comprises magnetic beads.

The magnetic beads within the vessel may be manipulated by a magnet positioned externally of the vessel.

Step (e) of the method may be effected in a number of ways.

Thus, for example, a magnet may be used to "hold" the magnetic beads (with immobilised biomolecule) at the side of the vessel. In the case of the preferred biomolecule purification assembly in which the vessel has a reduced diameter bore section, it is preferred that the beads are "held" at this position in the vessel.

Subsequently, the solution may be discharged from the vessel, wash buffer introduced into the vessel, and the magnet manipulated to aid re-suspension of the beads in the buffer. The magnet may once again be used to hold the beads in place and the wash buffer discharged from the vessel.

The washing operation may be repeated at least once. For preference the final wash step employs an aqueous solution of ethanol (preferably containing more than 60%v/v ethanol).

After removal of the final wash solution from the vessel, (and with the beads being held therein by means of the magnet), air may be passed over the beads to effect drying. Alternatively or additionally heat may be applied to the vessel, e.g. by locating the latter in a heating block.

Subsequently, the biomolecule may be eluted from the vessel. This may be achieved by introducing an elution buffer into the vessel, admixing the particles with the buffer, heating the admixture. immobilising the particles by means of the magnet, and discharging the solution (containing dissolved biomolecule) from the vessel for collection and subsequent processing

A particularly preferred embodiment of apparatus in accordance with the invention is capable of handling an array of biomolecule purification assemblies and therefore each of the individual steps (described above) of filtration, removal of the filter unit, "pick up" of magnetic particles, washing and elution is effected simultaneously on all members of the array.

In such an apparatus, it is preferred that the vessels (of the biomolecule purification assemblies) arc vertically disposed, open-ended columns. The upper ends of such columns may be associated with pumps for applying reduced pressure to the interior of the column for drawing liquid into the columns (e.g. for the purposes of the filtration and washing and elution operations) and for blowing or drawing drying air through the columns. Furthermore, the upper ends of the vessels may be associated, via appropriate valving arrangements, with appropriate reagent reservoirs permitting reagents to be passed downwardly into the columns if required.

A particularly preferred embodiment of apparatus in accordance with the invention for use in conjunction biomolecule purification assemblies as defined in the previous paragraph comprises separate filtration, bead "pick up", washing and elution stations Thus. at the filtration station. there may be a first set of wells (e.g. a BioBlock) each containing an aliquot of the suspension and each member of the array effects filtration of an aliquot from the corresponding well at the station. The apparatus may be such that the filter unit of the biomolecule purification assembly is discharged into the respective well at the filtration station. At the bead "pick up" station, there may be a second set of microtitre wells each containing magnetic beads in a solution of a binding agent. At the wash station, wash solution may be passed to the vessel (e.g. by pumping) and discharged through the liquid inlet into a waste receptacle which may be connected to a drain. Furthermore, the elution station may have a second set of microtitre wells containing elution buffer which is taken into the column and mixed with the beads. The final step of elution is then effected by holding the magnetic beads in position and discharging the liquid containing the desorbed biomolecule from the vessel. Obviously, the apparatus will include a magnet or magnets as necessary for manipulating the magnetic beads.

In a particularly preferred embodiment of the apparatus as described in the previous paragraph, the apparatus incorporates a head arrangement capable of selectively "picking-up" and releasing 12 of the biomolecule purification assemblies. Furthermore, the wells at the various stations may each be provided by a 12 by 8 array of microtitre wells so that in any one cycle of the apparatus a total of 12 samples may be processed. By operating the apparatus through 8 cycles then a total of 96 samples may be processed before the microtitre wells need to be replaced.

It is also preferred that the apparatus incorporates an upstream station at which the head arrangement is capable of "picking-up" the biomolecule purification assemblies to be used in any one cycle of the apparatus and a final discharge station at which the vessels of the assemblies are discarded. Thus, if the head arrangement is capable of holding 12 biomolecule purification assemblies then 12 such assemblies are "picked-up" at the beginning of each cycle of the machine.

Such an apparatus will further comprise mechanisms for moving those parts of the apparatus as are required to complete the method of the invention in the directions (X, Y and/or Z) so to do. The apparatus as described above may readily be automated and is capable of operating under the control of a programmed microprocessor.

It will thus be appreciated that a preferred embodiment of apparatus will comprise at least the features of
(a) a head arrangement having a plurality of individual column supporting heads on which the upper ends of disposable columns may be removably mounted and which have fluid flow passageways for transfer of fluids into and out of the upper ends of the columns;
(b) means for moving a supply of disposable columns so that upper ends of columns to be mounted on the supporting heads are presented below said heads;
(c) means for moving the head arrangement relatively downwardly towards the tops of the columns whereby the upper ends thereof become removably mounted on said head, and for moving the head arrangement relatively upwardly with columns mounted thereon in readiness for subsequent stages of the process.
(d) reservoirs for reagents/wash solutions as appropriate;
(e) means (e.g. for providing pressure variation in the columns) for causing liquid samples, liquid extracts or mixtures of such liquids with reagents thereof, provided at the lower tips of the columns (e.g. in wells of a microlite plate. BioBlock or similar) to be drawn upwardly into the columns and discharged from the lower tips thereof for processing steps as required to obtain the desired biomolecule; and
(f) means for removing the columns from the heads for disposal.

Such an apparatus is referred to herein as an "apparatus of the kind defined".

The apparatus of the kind defined may be operated in accordance with the sequence of processing steps as described more fully above. As a variation of that sequence (in which the disposable column is mounted on the column supporting head with removable filter attached to the column) the disposable column may initially be mounted on the supporting head and the filter subsequently mounted in the lower end of the column using the means (b) and (c) operated as appropriate.

The method and apparatus of the invention are particularly suitable for obtaining a sample of a biomolecule from a suspension obtained by a lysis procedure (e.g. a standard alkaline lysis procedure as well known to those skilled in the art) effected on a biological material.

The target biomolecule may, for example, be a nucleic acid (DNA or RNA) and may for example be a semi-purified or non purified, native or synthesised nucleic acid. The target soluble biomolecule may be any DNA or RNA sequence from a viral, bacterial, animal or plant source. Apart from the utility in purifying DNA and RNA samples and especially for purifying plasmid DNA and other recombinant DNA constructs, such as phagemids, free from chromosomal DNA, the method and apparatus according to the present invention are also suitable for isolating recombinant proteins and antibodies, especially from cellular samples

The biological material on which the lysis is effected may for example comprise cells. For the purposes of the present specification, the term "cell" is intended to encompass bacterial cells, cells (e.g. blood cells) from higher organisms, virus particles and other cell types or organelles which contain the target biomolecule and which may be released in a soluble form by a lysis procedure.

In the case of bacteria, the nucleic acid to be isolated may be from the bacterial genome or from an extragenomic element such as a plasmid. Phage infected bacteria may also be used for the preparation of phage DNA, such as M 13 DNA.

According to a preferred embodiment of the present invention the composition from which the target biomolecule is to be isolated is a lysed and neutralised bacterial cell composition containing soluble plasmid DNA and insoluble precipitated genomic DNA, flocculated protein and other cellular debris.

In order to isolate plasmid DNA that has been propagated within bacterial hosts, the following steps may be followed:
i) growth of bacterial host in an enriched medium;
ii) centrifugation of bacteria to form a pellet after which the supernatant is discarded;
iii) resuspension of the bacterial pellet in a buffered solution;
iv) addition of lysis reagent which releases the cellular contents; and
v) addition of neutralisation solution which causes the formation of suspension comprising a solution containing dissolved plasmid.

In a particularly advantageous implementation of the invention, the resuspension obtained from step (iii) may be provided, e.g. in a microtitre well, to the apparatus which is adapted to be such as to add lysis solution to the resuspension. In the preferred embodiment of the invention, in which the vessels (of the biomolecule purification assemblies) are vertically disposed, open-ended columns, the apparatus comprises a 'column-head' on which the biomolecule purification assemblies are to be mounted. However, prior to mounting of the assemblies on their respective heads, lysis solution may be injected ("fired") from the head into the resuspension in the micro-titre well. The neutralising solution may be added in the same way. This will also provide for good mixing of the lysis solution and neutralising solution with the resuspension. Subsequently, the biomolecule purification assemblies are mounted on their respective heads for effecting separation, washing and elution procedures as described.

In an alternative implementation, the lysis solution (drawn from a reservoir thereof) may be ejected downwardly through the column into the resuspension prior to mounting of the filter unit on the column.

In both cases the inventors have found that introduction of the lysis solution and neutralising solution in this way into the resuspension can avoid the need for any further mixing provided that sufficient time is allowed for lysis and neutralisation to be effected. Alternatively agitation may be employed (e.g. a shaking platform) to speed lysis/neutralisation. Furthermore, the introduction of the lysis and neutralising solution is effected without the dispensing apparatus coming into contact with the sample or its containing vessel.

Either single or multiple aliquots of lysis solution and neutralising solution may be discharged into each well. In a particularly convenient form of the apparatus, there is a single row of n (e.g. 12) column supporting heads and the cell samples to be processed arc held in wells of a microtitre plate or similar in which the wells are arranged as m rows each of n wells (i.e. an m x n array, e.g. 8 x 12). The use of multiple aliquots of lysis solution or neutralising solution will provide for better mixing (or at least more efficient extraction of products) than a single addition of reagent. In such an apparatus, the samples in the n wells of any one of the m rows are simultaneously treated with lysis reagents (by downward ejection from the n supporting heads). It will generally be preferred that the lysis reagents are discharged into all wells of the (m x n) array prior to the disposable columns being mounted on the heads for subsequent steps of the extraction procedure. Thus, lysis agents may initially be added to the n wells of the first of the m rows of the microtitre plate or similar, subsequently the plate is moved relative to the heads so that the second of the m rows is below the heads tor introduction of lysis reagents into these wells and so on until the m rows of wells have been treated with the lysis solution. If desired, the above procedure may then be repeated for the introduction of neutralising solution (via the heads) into the wells. The disposable columns may then be mounted on the supporting heads and all steps of the extraction procedure effected for the samples in the first row of wells prior to replacement of the columns, processing of the second row and so on until all wells of the (m x n) array have been processed.

In a modification of the procedure. columns may be mounted on the heads prior to dispense of the lysis and/or neutralisation reagents.

The time for which the lysis reagents are dispensed into each of the wells may for example be 0.1 to 1 seconds although this will depend in volume to be dispensed (typically 150µl of lysis solution and 150µl neutralisation solution) and pump speed. The discharge outlet of the column supporting head may be about 40mm to 100mm (preferably about 70mm) above the surface of the sample into which the lysis reagents are to be discharged. Typically also the discharge outlet of each supporting head will have a diameter of 0.1 to 1 mm (preferably about 0.75mm).

It should however be noted that all of the values set out in the previous paragraph are exemplary and other values, which may readily be evaluated, may be required depending on the actual construction of the apparatus.

The invention will be further described, by way of example only, with reference to the accompanying drawings, in which:
Fig. 1 schematically illustrates a first embodiment of the invention;
Fig 2 schematically illustrates the column of the assembly shown in Fig 1;
Fig 3 illustrates the sleeve of the filter unit in Fig 1;
Fig. 4 schematically illustrates to an enlarged scale the filter of the assembly shown in Fig 1;
Fig 5 is an exploded view, to an enlarged scale, of a biomolecule purification assembly incorporating a filter unit in accordance with a further embodiment of the invention;
Fig 6 illustrates to a still further enlarged scale, the filter incorporated in the filter unit illustrated in Fig 5;
Fig 7 illustrates a modification of the filter unit shown in Fig 6; and
Figs 8 and 9 schematically illustrate one embodiment of process in accordance with the invention.

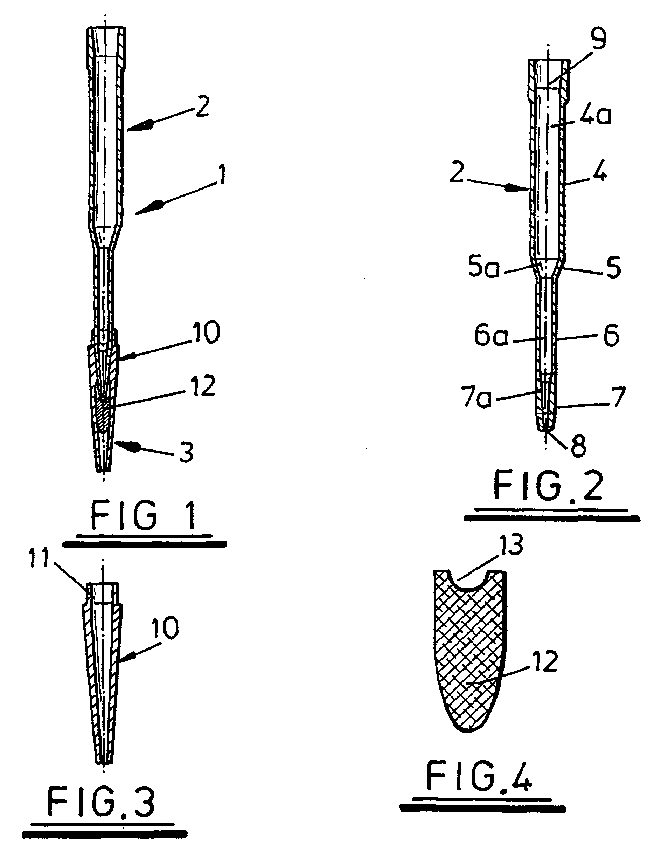

Referring firstly to Fig. 1, there is illustrated a biomolecule purification assembly 1 for use in obtaining a purified sample of a biomolecule of interest from a suspension (e.g. as obtained by an alkaline lysis procedure) comprising a solution of the biomolecule containing insoluble biological debris. The illustrated assembly 1 comprises a vertically disposed, open-ended column 2 and a filter unit 3.

Referring to Fig 2, column 2 is referenced for convenience as being comprised of body sections 4, 5, 6 and 7. Body section 4 defines an upper cylindrical bore 4a which at its lower end is connected to a downwardly tapering section 5a leading into a lower bore 6a which is of reduced diameter as compared to bore 4a. At its lower end bore 6a leads into a tapering section 7a defined within the lower section 7 of the column 2.

The lower end of tapering section 7a defines a liquid inlet 8 for the column.

At the upper end of column 2 there is provided a formation 9 by means of which the column may be mounted on the head of a sample processing apparatus of the type described more fully above.

Referring back to Fig 1, the filter unit 3, is a two component part and comprises a sleeve 10 (see Fig 3) having an internal bore 11 within which is housed a filter 12(see Fig 4) having depression 13 in its upper surface. Filter 11 is such that it is permeable to liquids but is capable of filtering the insoluble debris in the suspension from which the biomolecule is to be obtained.

Sleeve 10 is removably mounted on column 2 and more particularly is located over section 7 and the lower end of section 6 thereof. With the filter unit 3 located in position, the lowermost portion of section 7 of column 2 locates in the depression 13 in the upper surface of the filter 12 allowing the filtrate to come into proximity with the liquid inlet 8. As a result, the rate of filtration is enhanced.

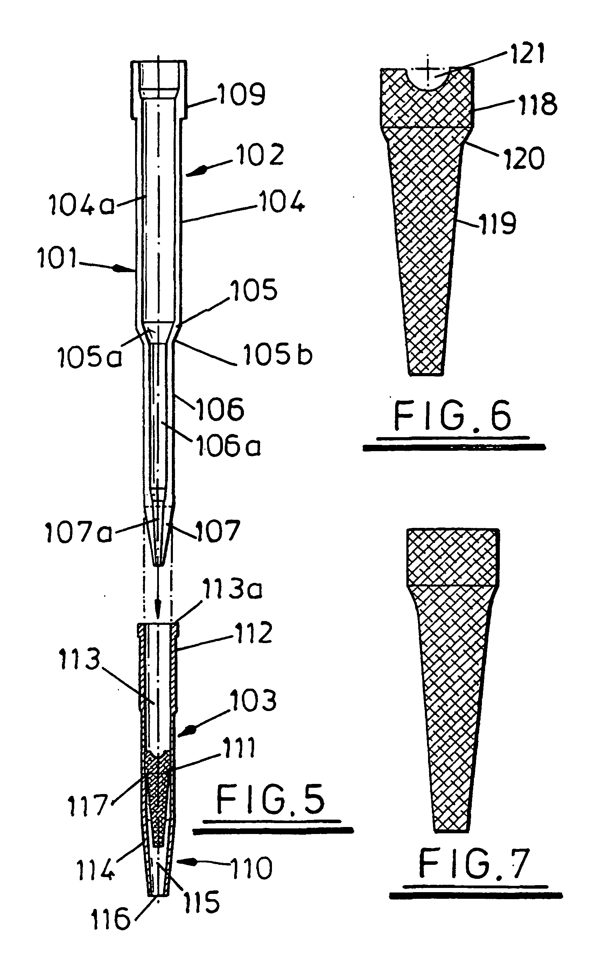

Referring to Fig 5, there is illustrated a biomolecule purification assembly 101 for use in obtaining a purified sample of a biomolecule of interest from a suspension (e.g. as obtained by an alkaline lysis procedure) comprising a solution of the biomolecule containing insoluble biological debris. The illustrated assembly 101 comprises a vertically disposed, open-ended column 102 and a filter unit 103.

The column 102 is referenced for convenience as being comprised of body sections 104, 105, 106 and 107. Body section 104 defines an upper cylindrical bore 104a which at its lower end is connected to a downwardly tapering section 105a leading into a lower bore 106a (in body section 106) which is of reduced diameter as compared to bore 104a. At its lower end bore 106a leads into a tapering section 107a defined within the lower section 107 of the column 102.

The lower end of tapering section 107a defines a filtrate inlet for the column.

A shoulder 105b is defined around body section 105.

At the upper end of column 102 there is provided a formation 109 by means of which the column may be mounted on the supporting head of a sample processing apparatus of the type described more fully above.

The filter unit 103 is a two component part and comprises a sleeve 110 within which is housed a filter 111.

The sleeve 110 has a first body portion 112 within which is defined a cylindrical bore 113 having an upper end 113a by means of which the filter unit 103 is mounted on the lower end of column 102. The second body portion 114 has an internal bore 115 which internally tapers from the first body portion 112 to a suspension inlet 116 of the filter unit 103.

At the transition of the bore 113 into bore 116 is a shoulder 117.

The filter 111 (see also Fig 5) comprises a cylindrical head portion 118 and frustoconical body portion 119. At the transition of head portion 118 into body portion 119 is a shoulder 120.

The filter unit 111 locates in sleeve 103 such that the head portion 118 (of the filter) occupies the full cross section of the bore 113 (of sleeve 103), the shoulder 120 (of filter 111) seats on the shoulder 117 (within sleeve 112) and the frustoconical body portion 119 (of the filter) extends into the second body portion 114 (of the sleeve 103) so as to taper therein towards its suspension inlet end 116.

The angle at which the frustoconcical body portion 119 (of the filter 111) tapers is greater than the angle of taper of the bore 115 (of the second body portion 114 of the sleeve 103) so that there is clearance between the outer surface of the frustoconical body portion 119 and the inner, tapering surface of the second body portion 114 of the sleeve 103. This arrangement ensures that there is a relatively large area of the filter exposed to the suspension to be filtered so that the filter unit has a high a capacity for filtration as possible given the overall dimensions of the filtration unit.

Furthermore, the "dead space" within the filter unit 103 (i.e. the space between the outer surface of the frustoconical body portion 119 of the filter 111 and the inner surface of the tapering bored 105) is relatively low whilst still allowing the advantage of the previous paragraph. This "low volume" dead space ensures that there is minimum liquid remaining in the dead space when all of the suspension to be filtered has been drawn up into the filter unit and air is about to enter the column.

At the top of the filter 111 is a generally hemispherical depression 121 which generally serves to improve the flow through the filter. In the assembled biomolecule purification unit, the filtrate inlet for the column 102 is level with the top of the hemispherical depression 121. The depression 121 is however optional and is omitted in the filter illustrated in Fig 7 which is otherwise identical to that of Fig 6.

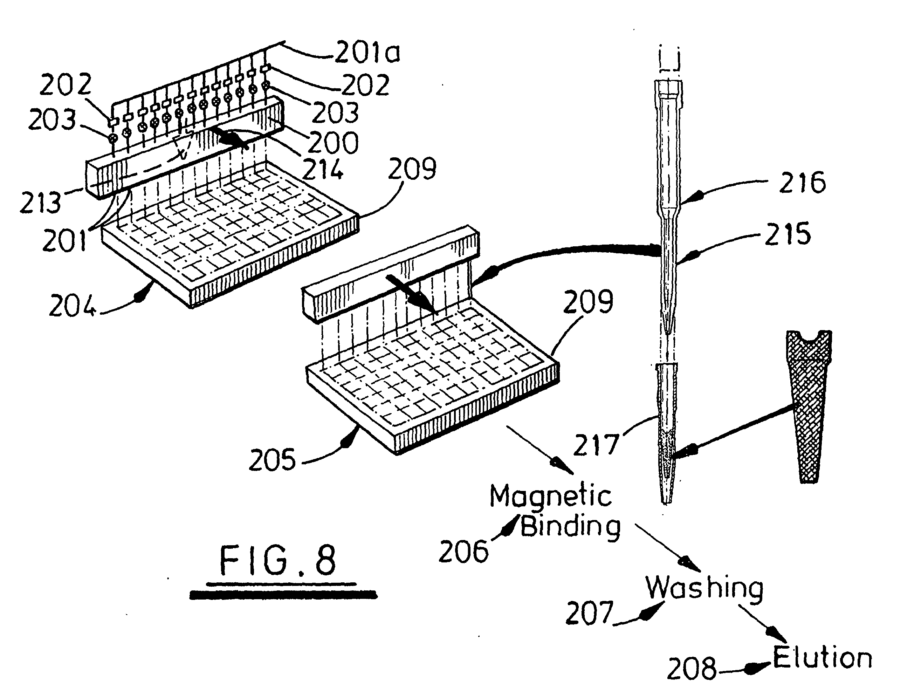

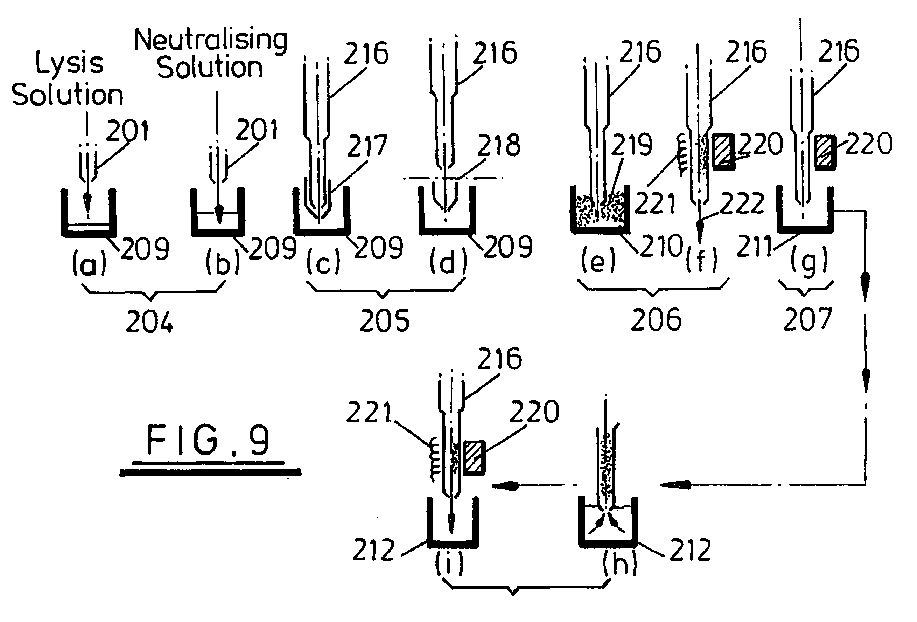

Reference is now made to Figs 8 and 9 which together schematically illustrate one embodiment of process in accordance with the invention.

As shown in Fig 8, the method is effected using an apparatus incorporating a head arrangement 200 having a line of twelve column supporting heads 201 each connected to a fluid supply line 201a via respectove pumps 202 and valves 203. The fluid supply line 201a is connected to a manifold (not shown) whereby the valves 203 which may selectively provide communication of the heads 201 with a vacuum pump or with liquid supply reservoirs (not shown).

Furthermore as shown in Fig 8, the method is shown as being effected at a number of sequential stations 204 to 208 designated as follows:

| Station | Function |
|---|---|
| 204 | Lysis/Neutralisation |
| 205 | Filtration |
| 206 | Magnetic Binding |
| 207 | Washing |
| 208 | Elution |

As described more fully below, a 12 x 8 BioBlock 209 is processed at each of stations 204 and 205. Furthermore 12 x 8 BioBlocks 210 to 212 are also provided at stations 206 to 208 respectively as described in detail below. For the purposes of simplicity only BioBlock 209 is illustrated in Fig 8, and single wells of all BioBlocks 209 to 212 are represented in Fig 9. The head arrangement 200 is illustrated as being movable upwardly and downwardly relataive to the BioBlocks 209 to 212 (as represented by arrow 213) and also relatively through the stations 204 to 208 as represented by arrow 214.

The detailed steps of the method will now be described with additional reference to Fig 9 which illustrates the individual steps of the method.

Station 204 is a lysis and neutralisation station. At this station there is provided the BioBlock 209 containing in each well thereof a sample to be lysed. The sample may for example be a suspension of bacteria containing a plasmid incorporating a DNA sequence of interest. Initially the head arrangement 200 is positioned above the first row of 12 wells (as shown in Fig 8) and is then lowered relatively thereto (see also Fig 9 (a)). Lysis solution provided in a liquid reservoir (not shown) is supplied through valves 203 so as to be "injected" downwardly and simultaneously into each of the twelve wells (of the first row) of the BioBlock. Each well may be treated with either a single aliquot of lysis solution or multiple aliquots thereof. Subsequently the head arrangement 200 is moved so as to supply lysis solution into each of the twelve wells of the second row of the BioBlock 209 and so on until all ninety six have had lysis solution added thereto. If necessary, the BioBlock 209 may be shaken so as to speed the lysis procedure. Subsequently head arrangement 200 is moved back to the first row of twelve wells (of the BioBlock 209). Neutralising solution, supplied via valves 203, is now passed though each of the heads 201 (again as either single or multiple aliquots) into the first row of twelve wells of the BioBlock 209 (as represented at Fig 9(b)). Subsequently the remaining wells of the BioBlock 209 are treated, twelve at a time, with the neutralising solution.

As the next step, biomolecule purification assemblies 215 (e.g. of the type illustrated as 1 in Fig 1 or 101 in Fig 5) are mounted on each of the column supporting heads 201. More particularly, each biomolecule purification assembly 215 is comprised of a column 216 and its separable filter unit 217. If the biomolecule of interest in the sample is DNA then columns may incorporate a solution of RNase to destroy RNA in the sample if this is likely to be an interferant in the analysis of DNA as isolated by the steps described below.

Filtration is shown as being effected at station 205 to which the head arrangement 200 and BioBlock 209 are moved from Station 204.

At the station 205. the head arrangement 200 is positioned such that the filter units 217 of each of the biomolecule purification assemblies 215 are immersed in the lysate contained in the first twelve rows of the BioBlock 209. Suction is then applied via valves 203 to the column supporting heads 201 whereby liquid is drawn upwardly into the column 216 but solids are prevented from doing so by the filter unit 217. A stripping comb 218 is now moved into position (see Fig 9 (d)) and head 200 is raised so that the filter 217 is removed from the lower end of column 216 and remains in the well of the BioBlock 209.

In the next step of the method, the head arrangement 200 (supporting the twelve columns 216 each containing filtered lysate held in the column by reduced pressure) is moved to station 206 at which there is a further BioBlock 210 containing in each well thereof a suspension of magnetic particles 219 in a binding medium. Head arrangement 200 is moved relatively downwardly so that the lower ends of the columns 215 are immersed in the suspension of the magnetic particles (see Fig 9(e)). The filtered lysate in the columns 216 is now discharged into the wells of BioBlock 210 for mixing with the suspension the magnetic particles 219 and the mixture is then taken back up into the column 216. There may be at least one subsequent cycle of discharge of this mixture back in to the well of the BioBlock 210 and take up of the mixture back into column 216 to ensure thorough mixing.

A magnet 220 with associated heater 221 is then moved into position. The magnet 220 ensures that the magnetic particles 218 are retained at one position in the column 215 and held at elevated temperature (e.g. 45°C) for a few seconds, e.g. 10 seconds.

The biomolecule of interest will by now have become bound to the particles 219 so that the liquid remaining in column 216 may be discharged therefrom effectively as waste as represented by arrow 222 (Fig 9(f)).

The head arrangement 200 is next moved to station 207 at which a wash solution is introduced via valves 203 into the top of columns 216 so as to wash the particles 219 down into the wells of BioBlock 207. The mixture of particles 219 and wash solution (e.g. 70% aqueous ethanol) may be subjected to repeat cycles of aspiration back into the column and discharge into the wells of BioBlock 207 so as to ensure thorough washing of the particle 219. The washed particles (with biomolecule bound thereto) are then retained in column 216 and the wash solution discharged into the well of BioBlock 211 (Fig 9(g)).

Finally. head arrangement is moved to station 208 where there is a further BioBlock 212 containing an eluant (e.g. sterile water) which is aspirated into the column of admixture with the particles 219 (released from magnet 220) and release of the bound biomolecule therefrom (Fig 9(h)). During this step the heater 221 may be heated to 60°C. Subsequently the magnet 220 is re-applied and eluant, containing the dissolved biomolecule of interest. discharged into the BioBlock 209 (Fig 9(i).

The twelve columns 216 are now released from the column supporting heads 201 and are discharged to waste (not shown). Subsequently a new set of biomolecule purification assemblies 215 are mounted on the head arrangement 200 which is then positioned over the second row of 12 wells in BioBlock 209 at filtration station 205. The steps described above in relation to Figs 9(c)-(i) are then repeated so as to obtain further samples of the purified biomolecules. Further repeats of this procedure are effected until all ninety six wells of the BioBlock 209 have been processed.

For the purposes of describing the steps of the above process, it has been assumed that head arrangement 200 is translated in the direction of arrow 214 between the various stations 204 to 208. However, in practice, it is possible for there to be no transitional movement in the direction of arrow 214 of the head arrangement 200 but rather for the BioBlocks 209 to 212 to be moved beneath the head arrangement 200 as appropriate.

## Claims

1. A method of obtaining a sample of a biomolecule from a suspension comprising a solution containing the biomolecule and insoluble material, the method comprising the steps of:
(a) providing a biomolecule purification assembly comprised of a vessel having a liquid inlet and of a filter unit removably located on the liquid inlet;
(b) effecting a filtration of the suspension through the filter unit so as to cause the solution to enter the vessel through the liquid inlet;
(c) removing the filter unit from the liquid inlet;
(d) immobilising the biomolecule on a solid phase support; and
(e) subjecting the biomolecule to at least one of the steps of washing on the support and elution from the support to obtain a purified sample of the biomolecule.

2. A method as claimed in claim 1 wherein the biomolecule is a nucleic acid.

3. A method as claimed in claim 2 wherein the nucleic acid is DNA.

4. A method as claimed in claim 3 wherein the DNA is plasmid DNA.

5. A method as claimed in any one of claims 1 to 4 wherein the step (e) comprises washing the solid phase support and said support is contained within the vessel during this step.

6. A method as claimed in claim 5 wherein step (e) comprises eluting the biomolecule from solid phase support contained within the vessel, the elution being effected through said inlet of the vessel.

7. A method as claimed in any one of claims 1 to 6 wherein the step (b) the suspension to be filtered is contained in a well and in step (c) the filter unit is discharged into that well.

8. A method as claimed in any one of claim 1 to 7 wherein the biomolecule is absorbed onto the solid phase support in the presence of a binding agent for effecting said adsorption.

9. A method as claimed in claim 8 wherein, for the purposes of step (d) the filtrate in the vessel contains the binding agent and the filtrate is discharged onto the support and the resultant mixture is taken back into the vessel.

10. A method as claimed in claim 8 wherein, for the purposes of step (d), the filtrate is discharged into a mixture of the support and the binding agent and the resultant mixture is taken back into the vessel.

11. A method as claimed in any one of claims 1 to 10 wherein the solid phase support comprises magnetic beads.

12. A method as claimed in any one of claims 1 to 11 wherein the vessel of the biomolecule purification assembly is an open-ended, vertically disposed column.

13. A method as claimed in claim 12 wherein the column has an upper bore section and a lower bore section of reduced diameter as compared to the upper section.

14. Apparatus for obtaining a sample of a biomolecule from a suspension comprising a solution of the biorriolecule and insoluble material the apparatus comprising
(i) a filtration station at which is provided a biomolecule purification assembly comprised of a vessel having a liquid inlet and a removable filter unit located at the inlet of the vessel, said filtration station being provided with means for causing the solution present in the suspension to pass through the filter unit and the liquid inlet into the vessel;
(ii) means for removing the filter unit from the vessel; and
(iii) at least one of a washing station and an elution station.

15. Apparatus as claimed in claim 14 comprising an elution station and a washing station.

16. Apparatus as claimed in claim 14 or 15 further comprising a solid phase support supply station preferably provided between (ii) and (iii).

17. An apparatus as claimed in any one of claims 14 to 16 wherein the vessel is an open-ended flow-through column.

18. An apparatus as claimed in claim 17 wherein the column has an upper bore section and a lower bore section of reduced diameter compared to the upper section.

19. An apparatus as claimed in claim 17 or 18 wherein the filter unit comprises a sleeve which incorporates the filter and which is located over the lower end of the column.

20. An apparatus as claimed in claim 19 wherein the upper surface of the filter has a depression and the lower end of the column locates in the depression.

21. An apparatus as claimed in any one of claims 14 to 20 in which the pore size of the filter is in the range 0.2 to 50 microns.

22. An apparatus according to any one of claims 14 to 21 in which the suspension to be filtered is contained in a well and once the filtration operation is complete the filter unit is automatically discarded into that well.

23. An apparatus according to claim 22 further comprising a stripping arrangement which acts on the filter unit when the assembly is moved upwards causing the filter unit to be discharged into the well.

24. An apparatus according to any one of claims 14 to 23 which is capable of handling an array of biomolecule purification assemblies.

25. An apparatus according to claim 24 wherein each individual step of filtration, removal of the filtration unit washing and elution is adapted to be effected simultaneously on all members of the array.

26. An apparatus as claimed in claim 14 comprising
(a) a head arrangement having a plurality of individual column supporting heads on which the upper ends of disposable columns may be removably mounted and which have fluid flow passageways for transfer of fluids into and out of the upper ends of the columns;
(b) means for moving a supply of disposable columns so that upper ends of columns to be mounted on the supporting heads are presented below said heads;
(c) means for moving the head arrangement relatively downwardly towards the tops of the columns whereby the upper ends thereof become removably mounted on said head, and for moving the head arrangement relatively upwardly with columns mounted thereon in readiness for subsequent stages of the process.
(d) reservoirs for reagents/wash solutions as appropriate;
(e) means (e.g. for providing pressure variation in the columns) for causing liquid samples, liquid extracts or mixtures of such liquids with reagents thereof, provided at the lower tips of the columns (e.g. in wells of a microlite plate, BioBlock or similar) to be drawn upwardly into the columns and discharged from the lower tips thereof for processing steps as required to obtain the desired biomolecule; and
(f) means for removing the columns from the heads for disposal.

27. A biomolecule purification assembly comprising a vessel having a liquid inlet and a filter unit removably located on the liquid inlet wherein the vessel is in the form of a column having a first bore section and a second bore section of reduced diameter as compared to the first section, and the filter unit is comprised of a sleeve which houses the filter and which is removably located on the end of the vessel remote from the first bore section.

28. A biomolecule purification assembly as claimed in claim 27 wherein the surface of the filter adjacent to the inlet of the vessel has a depression and the end of the vessel locates in the depression.

29. A filter unit for a biomolecule purification assembly according to claim 27 or 28, said filter unit comprising
(a) an elongate sleeve having one end (the "suspension inlet end") through which a suspension to be filtered enters the sleeve and an opposite end for location over a column of a biomolecule punncation assembly, said sleeve having a first body portion and a second body portion which internally tapers from the first body portion to said suspension inlet end, and
(b) a filter having a head portion locating in, and occupying the cross-section of, the first body portion, and a frustoconical or conical body portion extending into said second body portion of the sleeve and tapering therein at a larger angle than the internal taper of the sleeve, and the filter unit being removably located in said assembly.

30. A biomolecule purification assembly according to claim 27 or 28 comprising
(i) an elongate vessel in the form of a column having first and second open ends with a filtrate inlet being provided at the first end of the column, and
(ii) the filter unit as defined in claim 29 removably located over the first end of the vessel.

31. An assembly as claimed in claim 30 wherein the filter unit has an upper depression and said filtrate inlet of the elongate vessel is located in the region of said depression.

32. An assembly as claimed in claim 30 or 31 wherein said elongate vessel is as defined in claim 24.

33. A method as claimed in claim 1 wherein the suspension is obtained by the steps of:
(i) providing a re-suspension of a bacterial pellet in solution;
(ii) adding lysis solution to the re-suspension to effect release of cellular contents; and
(iii) adding neutralising solution to the lysed re-suspension to effect the formation of a suspension comprising a solution of a biomolecule and insoluble material for use in the method of claim 1;
in which the lysis solution and/or neutralising solution are each added in multiple aliquots which are ejected downwardly into the re-suspension to provide the suspension in the absence of additional mechanical agitation or stirring without the dispensing apparatus coming into contact with the sample or the containing vessel.

34. A method of operating an apparatus as defined in claim 26 using the steps of lysing the cell sample and obtaining the biomolecule from the lysate wherein reagents for lysis are ejected downwardly through the supporting heads into the cell sample to be lysed (either prior or subsequent to mounting columns on the heads).

35. A method as claimed in claim 34 wherein the lysis reagents comprise a lysis solution and a separate neutralising solution.

36. A method as claimed in any one of claims 33 to 35 wherein the cell samples to be lysed are contained within the individual wells of a microtitre well tray or the like and the lysis reagents are ejected into the individual wells.

37. A method as claimed in claim 36 wherein the apparatus comprises a single row of n column supporting heads and the cell samples to be processed are provided in an (m x n) array of microtitre wells wherein the samples in the n wells of any one of the m rows are simultaneously treated with lysis reagents by downward ejection from the n supporting heads.

38. A method as claimed in claim 37 wherein the lysis reagents are discharged into all wells of the (m x n) array prior to disposable columns being mounted on the heads for subsequent steps of the extraction procedure.

39. A method as claimed in any one of claims 33 to 38 wherein the time of dispense for the lysis reagent into a cell sample is 0.1 to 1 second.

40. A method as claimed in any one of claims 33 to 39 wherein the discharge outlet of the column supporting heads is 40mm to 100mm above the surface of the sample into which the lysis reagents are to be discharged.

41. A method as claimed in claim 40 wherein said discharge outlet is about 70mm above said surface of the sample.

42. A method as claimed in any one of claims 33 to 41 wherein the discharge outlet of each supporting head has a diameter of 0.1 to 1mm.

43. A method as claimed in claim 42 wherein said diameter is about 0.75mm.

## Patentansprüche

1. Verfahren zur Entnahme einer Probe eines Biomoleküls aus einer Suspension umfassend eine Lösung, die das Biomolekül und unlösliches Material enthält, wobei das Verfahren die folgenden Schritte umfasst:
(a) Bereitstellen eines Aufbaus zur Reinigung von Biomolekülen, bestehend aus einem Behälter mit einem Flüssigkeitseinlass und einer Filtereinheit, die sich entfembar am Flüssigkeitseinlass befindet;
(b) Bewirken einer Filtration der Suspension durch die Filtereinheit dergestalt, um die Lösung zu veranlassen, durch den Flüssigkeitseinlass in den Behälter einzutreten;
(c) Entfernen der Filtereinheit vom Flüssigkeitseinlass;
(d) Immobilisieren des Biomoleküls auf einem Festphasenträger; und
(e) Aussetzen des Biomoleküls mindestens einem der Schritte Waschen auf dem Träger und Elution aus dem Träger zum Erhalt einer gereinigten Probe des Biomoleküls.

2. Verfahren nach Anspruch 1, worin das Biomolekül eine Nukleinsäure darstellt.

3. Verfahren nach Anspruch 2, worin die Nukleinsäure DNA darstellt.

4. Verfahren nach Anspruch 3, worin die DNA eine Plasmid-DNA darstellt.

5. Verfahren nach einem der Ansprüche 1 bis 4, worin der Schritt (e) das Waschen des Festphasenträgers umfasst und der Träger während dieses Schrittes im Behälter enthalten ist.

6. Verfahren nach Anspruch 5, worin Schritt (e) das Eluieren des Biomoleküls aus dem im Behälter enthaltenen Festphasenträger umfasst, wobei die Elution durch den Einlass des Behälters bewirkt wird.

7. Verfahren nach einem der Ansprüche 1 bis 6, worin im Schritt (b) die zu filtrierende Suspension in einer Vertiefung enthalten ist und in Schritt (c) die Filtereinheit in diese Vertiefung abgegeben wird.

8. Verfahren nach einem der Ansprüche 1 bis 7, worin das Biomolekül in Gegenwart eines Bindemittels zur Bewirkung der Adsorption an den Festphasenträger absorbiert wird.

9. Verfahren nach Anspruch 8, worin zu den Zwecken von Schritt (d) das Filtrat im Behälter das Bindungsmittel enthält und das Filtrat an den Träger abgegeben wird und das resultierende Gemisch zurück in den Behälter geleitet wird.

10. Verfahren nach Anspruch 8, worin für die Zwecke von Schritt (d) das Filtrat in ein Gemisch aus dem Träger und dem Bindemittel abgegeben wird und das resultierende Gemisch zurück in den Behälter geleitet wird.

11. Verfahren nach einem der Ansprüche 1 bis 10, worin der Festphasenträger Magnetperlen umfasst.

12. Verfahren nach einem der Ansprüche 1 bis 11, worin der Behälter des Aufbaus zur Reinigung von Biomolekülen eine offenendige, vertikal angeordnete Säule darstellt.

13. Verfahren nach Anspruch 12, worin die Säule einen oberen Bohrungsabschnitt und einen unteren Bohrungsabschnitt von reduziertem Durchmesser im Vergleich zum oberen Abschnitt aufweist.

14. Apparat zur Entnahme einer Probe eines Biomoleküls aus einer Suspension, umfassend eine Lösung aus dem Biomolekül und unlöslichen Material, wobei der Apparat Folgendes wnfasst:
(i) eine Filtrationsstation, an der ein Aufbau zur Reinigung von Biomolekülen bereitgestellt ist, die aus einem Behälter mit einem Flüssigkeitseinlass und einer sich am Einlass des Behälters befindenden entfernbaren Filtereinheit besteht, wobei die Filtrationsstation mit Mitteln zum Veranlassen der in der Suspension vorliegenden Lösung, durch die Filtereinheit und den Flüssigkeitseinlass in den Behälter zu passieren, versehen ist;
(ii) Mittel zum Entfernen der Filtereinheit aus dem Behälter; und
(iii) mindestens eine von einer Waschstation und einer Elutionsstation.

15. Apparat nach Anspruch 14, umfassend eine Elutionsstation und eine Waschstation.

16. Apparat nach einem der Ansprüche 14 oder 15 weiter umfassend eine Festphasenträger-Versorgungsstation, die bevorzugt zwischen (ii) und (iii) bereitgestellt ist.

17. Apparat nach einem der Ansprüche 14 bis 16, worin der Behälter eine offenendige Durchflusssäule darstellt.

18. Apparat nach Anspruch 17, worin die Säule einen oberen Bohrungsabschnitt und einen unteren Bohrungsabschnitt von reduziertem Durchmesser im Vergleich zum oberen Abschnitt aufweist.

19. Apparat nach Anspruch 17 oder 18, worin die Filtereinheit eine Hülse umfasst, welche das Filter enthält und welche sich über dem unteren Ende der Säule befindet.

20. Apparat nach Anspruch 19, worin die obere Oberfläche des Filters eine Mulde aufweist und sich das untere Ende der Säule in der Mulde befindet.

21. Apparat nach einem der Ansprüche 14 bis 20, worin sich die Porengröße des Filters im Bereich von 0,2 bis 50 Mikron befindet.

22. Apparat nach einem der Ansprüche 14 bis 21, worin die zu filtrierende Suspension in einer Vertiefung enthalten ist und sobald der Filtrationsvorgang abgeschlossen ist, die Filtereinheit automatisch in diese Vertiefung abgegeben wird.

23. Apparat nach Anspruch 22, weiter umfassend eine Strippanordnung, die auf die Filtereinheit wirkt, wenn der Aufbau nach oben bewegt wird, wobei die Filtereinheit veranlasst wird, in die Vertiefung abgegeben zu werden.

24. Apparat nach einem der Ansprüche 14 bis 23, der zur Handhabung einer Anordnung von Aufbauten zur Reinigung von Biomolekülen fähig ist.

25. Apparat nach Anspruch 24, worin jeder einzelne Schritt zur Filtration, Entfernung der Filtrationseinheit, zum Waschen und zur Elution angepasst ist, um simultan an allen Elementen der Anordnung bewirkt zu werden.

26. Apparat nach Anspruch 14, umfassend:
(a) eine Kopfanordnung mit einer Vielzahl individueller Säulenträgerköpfen, auf denen die oberen Enden der Einwegsäulen entfernbar befestigt werden können und die Passagewege für den Flüssigkeitsfluss zum Transfer von Flüssigkeiten in und aus den oberen Enden der Säulen aufweisen;
(b) Mittel zum Bewegen einer Zufuhr von Einwegsäulen, so dass die oberen Enden der an den Trägerköpfen zu befestigenden Säulen unter den Köpfen dargestellt sind;
(c) Mittel zur Bewegung der Kopfanordnung relativ nach unten in Richtung der Oberseiten der Säulen, wobei die oberen Enden davon entfernbar am Kopf befestigt werden und zur Bewegung der Kopfanordnung relativ nach oben, wobei die Säulen daran in Bereitschaft der sich anschließenden Verfahrensstufen befestigt sind;
(d) gegebenenfalls Reservoire für Reagenzien/Waschlösungen;
(e) Mittel (z. B. zur Bereitstellung von Druckvariation in den Säulen) zum Veranlassen, dass Flüssigkeitsproben, Flüssigkeitsextrakte oder Gemische solcher Flüssigkeiten mit Reagenzien davon, die an den unteren Spitzen der Säulen (z. B. in Vertiefungen einer Microlite-Platte, BioBlock oder ähnlich) bereitgestellt werden, nach oben in die Säulen gezogen und von den unteren Spitzen davon gegebenenfalls für Verarbeitungsschritte zum Erhalt des gewünschten Biomoleküls abgegeben werden; und
(f) Mittel zur Entfernung der Säulen aus den Köpfen zur Entsorgung.

27. Aufbau zur Reinigung von Biomolekülen, umfassend einen Behälter mit einem Flüssigkeitseinlass und einer Filtereinheit, die sich entfernbar am Flüssigkeitseinlass befindet, worin der Behälter in der Form einer Säule mit einem ersten Bohrungsabschnitt und einem zweiten Bohrungsabschnitt von reduziertem Durchmesser im Vergleich zum ersten Abschnitt vorliegt und die Filtereinheit aus einer Hülse besteht, in der das Filter untergebracht ist und die sich entfernbar am Ende des Behälters vom ersten Bohrungsabschnitt entfernt befindet.

28. Aufbau zur Reinigung von Biomolekülen nach Anspruch 27, worin die Oberfläche des Filters an den Einlass des Behälters angrenzend eine Mulde aufweist und sich das Ende des Behälters in der Mulde befindet.

29. Filtereinheit für einen Aufbau zur Reinigung von Biomolekülen nach Anspruch 27 oder 28, wobei die Filtereinheit Folgendes umfasst:
(a) eine verlängerte Hülse mit einem Ende (dem "Einlassende der Suspension"), durch das eine zu filtrierende Suspension in die Hülse eintritt und ein entgegengesetztes Ende zur Platzierung über einer Säule eines Aufbaus zur Reinigung von Biomolekülen, wobei die Hülse einen ersten Körperteil und einen zweiten Körperteil aufweist, der sich intern vom ersten Körperteil zum Suspensionseinlassende verjüngt, und
(b) ein Filter, das einen Kopfteil aufweist und sich im ersten Körperteil befindet und dessen Querschnitt einnimmt, und einen stumpfkegeligen oder konischen Körperteil, der sich in den zweiten Körperteil der Hülse erstreckt und sich darin bei einem größeren Winkel als die interne Verjüngung der Hülse verjüngt und wobei sich die Filtereinheit entfernbar in dem Aufbau befindet.

30. Aufbau zur Reinigung von Biomolekülen nach Anspruch 27 oder 28, umfassend:
(i) einen verlängerten Behälter in der Form einer Säule mit ersten und zweiten offenen Enden mit einem Filtrateinlass, der am ersten Ende der Säule bereitgestellt ist, und
(ii) die Filtereinheit, wie nach Anspruch 29 definiert, die sich entfernbar über dem ersten Ende des Behälters befindet.

31. Aufbau nach Anspruch 30, worin die Filtereinheit eine obere Mulde aufweist und der Filtrateinlass des verlängerten Behälters sich im Bereich der Mulde befindet.

32. Aufbau nach Anspruch 30 oder 31 worin der verlängerte Behälter wie nach Anspruch 24 definiert ist.

33. Verfahren nach Anspruch 1, worin die Suspension durch die folgenden Schritte erhalten wird:
(i) Bereitstellung einer Resuspension eines Bakterienpellets in Lösung;
(ii) Zufügen von Lyselösung zur Resuspension zur Bewirkung der Freisetzung des Zellinhalts; und
(iii) Zufügen von Neutralisationslösung zur lysierten Resuspension zur Bewirkung der Bildung einer Suspension, umfassend eine Lösung aus einem Biomolekül und unlöslichem Material zur Verwendung im Verfahren nach Anspruch 1;
worin die Lyselösung und/oder Neutralisationslösung jeweils in multiplen Aliquoten zugefügt werden, die nach unten in die Resuspension zur Bereitstellung der Suspension in Abwesenheit von zusätzlicher mechanischer Rührung oder Rühren, ohne dass der Abgabeapparat mit der Probe oder dem die Probe enthaltenden Behälter in Kontakt kommt, ausgestoßen werden.

34. Verfahren zum Betreiben eines Apparates nach Anspruch 26 unter Verwendung der Schritte des Lysierens der Zellprobe und Erhalt des Biomoleküls aus dem Lysat, worin Reagenzien zur Lyse nach unten durch die Trägerköpfe in die zu lysierende Zellprobe (entweder vor oder anschließend an die Befestigung der Säulen an den Köpfen) ausgestoßen werden.

35. Verfahren nach Anspruch 34, worin die Lysereagenzien eine Lyselösung und eine getrennte Neutralisationslösung umfassen.

36. Verfahren nach einem der Ansprüche 33 bis 35, worin die zu lysierenden Zellproben in den individuellen Vertiefungen einer Mikrotiterschale mit Vertiefungen oder dergleichen enthalten sind, und die Lysereagenzien in die individuellen Vertiefungen ausgestoßen werden.

37. Verfahren nach Anspruch 36, worin der Apparat eine einzelne Reihe von n Säulenträgerköpfen umfasst und die zu verarbeitenden Zellproben in einer (m x n) Anordnung von Mikrotitervertiefungen bereitgestellt sind, worin die Proben in den n Vertiefungen von jedweder einen der m Reihen gleichzeitig mit Lysereagenzien mittels Ausstoßen aus den n Trägerköpfen nach unten behandelt werden.

38. Verfahren nach Anspruch 37, worin die Lysereagenzien in alle Vertiefungen der (m x n) Anordnung abgegeben werden, bevor die Einwegsäulen an den Köpfen für die anschließenden Schritte des Extraktionsverfahrens befestigt werden.

39. Verfahren nach einem der Ansprüche 33 bis 38, worin die Zeit der Abgabe für das Lysereagenz in eine Zellprobe bei 0,1 bis 1 Sekunde liegt.

40. Verfahren nach einem der Ansprüche 33 bis 39, worin sich der Abgabeauslass der Säulenträgerköpfe 40 mm bis 100 mm über der Oberfläche der Probe befindet, in welche die Lysereagenzien abgegeben werden sollen.

41. Verfahren nach Anspruch 40, worin sich der Abgabeauslass ca. 70 mm über der Oberfläche der Probe befindet.

42. Verfahren nach einem der Ansprüche 33 bis 41, worin der Abgabeauslass von jedem Trägerkopf einen Durchmesser von 0,1 bis 1 mm aufweist.

43. Verfahren nach Anspruch 42, worin der Durchmesser ca. 0,75 mm beträgt.

## Revendications

1. Procédé d'obtention d'un échantillon d'une biomolécule à partir d'une suspension comprenant une solution contenant la biomolécule et du matériau insoluble, le procédé comprenant les étapes consistant à:
(a) réaliser un assemblage de purification de biomolécule constitué d'un récipient possédant une entrée de liquide et d'une unité de filtrage située de manière amovible sur l'entrée de liquide;
(b) effectuer une filtration de la suspension à travers l'unité de filtrage de manière à forcer la solution à entrer dans le récipient à travers l'entrée de liquide;
(c) retirer l'unité de filtrage de l'entrée de liquide;
(d) immobiliser la biomolécule sur un support en phase solide; et
(e) soumettre la biomolécule à au moins une des étapes de lavage sur le support et d'élution à partir du support pour obtenir un échantillon purifié de la biomolécule.

2. Procédé comme revendiqué dans la revendication 1, dans lequel la biomolécule est un acide nucléique.

3. Procédé comme revendiqué dans la revendication 2, dans lequel l'acide nucléique est de l'ADN.

4. Procédé comme revendiqué dans la revendication 3, dans lequel l'ADN est de l'ADN de plasmide.

5. Procédé comme revendiqué dans une quelconque des revendications 1 à 4, dans lequel l'étape (e) comprend le lavage du support en phase solide et dans lequel ledit support est contenu à l'intérieur du récipient durant cette étape.

6. Procédé comme revendiqué dans la revendication 5, dans lequel l'étape (e) comprend l'élution de la biomolécule à partir du support en phase solide contenu à l'intérieur du récipient, l'élution étant effectuée à travers ladite entrée du récipient.

7. Procédé comme revendiqué dans une quelconque des revendications 1 à 6, où dans l'étape (b) la suspension à filtrer est contenue dans un puits et où dans l'étape (c) l'unité de filtrage est déchargée dans ce puits.

8. Procédé comme revendiqué dans une quelconque des revendications 1 à 7, dans lequel la biomolécule est absorbée sur le support en phase solide en présence d'un agent liant pour effectuer ladite adsorption.

9. Procédé comme revendiqué dans la revendication 8, dans lequel, pour les finalités de l'étape (d), le filtrat dans le récipient contient l'agent liant et le filtrat est déchargé sur le support et le mélange résultant est ramené dans le récipient.

10. Procédé comme revendiqué dans la revendication 8, dans lequel, pour les finalités de l'étape (d), le filtrat est déchargé en formant un mélange du support et de l'agent liant et le mélange résultant est ramené dans le récipient.

11. Procédé comme revendiqué dans une quelconque des revendications 1 à 10, dans lequel le support en phase solide comprend des perles magnétiques.

12. Procédé comme revendiqué dans une quelconque des revendications 1 à 11, dans lequel le récipient de l'assemblage de purification de biomolécule est une colonne disposée verticalement à extrémité ouverte.

13. Procédé comme revendiqué dans la revendication 12, dans lequel la colonne possède une section de conduite supérieure et une section de conduite inférieure de diamètre réduit comparé à la section supérieure.

14. Appareil pour l'obtention d'un échantillon d'une biomolécule à partir d'une suspension comprenant une solution de la biomolécule et du matériau insoluble, l'appareil comprenant
(i) une station de filtration qui est pourvue d'un assemblage de purification de biomolécule constitué d'un récipient possédant une entrée de liquide et d'une unité de filtrage amovible située à l'entrée du récipient, ladite station de filtration étant pourvue d'un moyen pour faire passer la solution présente dans la suspension à travers l'unité de filtrage et l'entrée de liquide de manière à ce qu'elle entre dans le récipient;
(ii) un moyen pour retirer l'unité de filtrage du récipient; et
(iii) au moins une station parmi une station de lavage et une station d'élution.

15. Appareil comme revendiqué dans la revendication 14 comprenant une station d'élution et une station de lavage.

16. Appareil comme revendiqué dans la revendication 14 ou 15 comprenant en outre une station d'approvisionnement de support en phase solide prévue préférablement entre (ii) et (iii).

17. Appareil comme revendiqué dans une quelconque des revendications 14 à 16, dans lequel le récipient est une colonne à écoulement continu et à extrémité ouverte.

18. Appareil comme revendiqué dans la revendication 17, dans lequel la colonne possède une section de conduite supérieure et une section de conduite inférieure de diamètre réduit comparé à la section supérieure.

19. Appareil comme revendiqué dans la revendication 17 ou 18, dans lequel l'unité de filtrage comprend un manchon qui incorpore le filtre et qui est situé sur l'extrémité inférieure de la colonne.

20. Appareil comme revendiqué dans la revendication 19, dans lequel la surface supérieure du filtre possède une dépression et l'extrémité inférieure de la colonne se situe dans la dépression.

21. Appareil comme revendiqué dans une quelconque des revendications 14 à 20, dans lequel la dimension de pore du filtre est dans le domaine de 0,2 à 50 microns.

22. Appareil selon une quelconque des revendications 14 à 21, dans lequel la suspension à filtrer est contenue dans un puits et une fois que l'opération de filtration est terminée l'unité de filtrage se décharge automatiquement dans ce puits.

23. Appareil selon la revendication 22 comprenant en outre un agencement de raclage qui agit sur l'unité de filtrage lorsque l'assemblage se déplace vers le haut en amenant l'unité de filtrage à se décharger dans le puits.

24. Appareil selon une quelconque des revendications 14 à 23 qui est capable de traiter un réseau d'assemblages de purification de biomolécule.

25. Appareil selon la revendication 24, dans lequel chaque étape individuelle de filtration, de retrait de l'unité de filtration, de lavage et d'élution est adaptée pour être effectuée simultanément sur tous les éléments du réseau.

26. Appareil comme revendiqué dans la revendication 14 comprenant
(a) un agencement de têtes possédant une pluralité de têtes de support de colonne individuelles sur lesquelles les extrémités supérieures des colonnes jetables peuvent être montées de manière amovible et qui possèdent des couloirs d'écoulement de fluide pour le transfert de fluides dans et hors des extrémités supérieures des colonnes;
(b) un moyen pour déplacer une réserve de colonnes jetables de sorte que les extrémités supérieures des colonnes à monter sur les têtes de support se présentent en dessous desdites têtes;
(c) un moyen pour déplacer l'agencement de têtes de manière relative vers le bas en direction des sommets des colonnes de sorte que leurs extrémités supérieures soient montées de manière amovible sur ledit agencement de têtes, et pour déplacer l'agencement de têtes de manière relative vers le haut avec des colonnes montées dessus en préparation des étapes ultérieures du procédé.
(d) des réservoirs pour des réactifs / des solutions de lavage si nécessaire;
(e) un moyen (p.ex. pour réaliser une variation de pression dans les colonnes) pour amener des échantillons liquides, des extraits liquides ou des mélanges de tels liquides avec leurs réactifs, qui se trouvent aux pointes inférieures des colonnes (p.ex. dans des puits d'une plaque en microlithe, BioBlock ou similaire) à être aspirés vers le haut dans les colonnes et déchargés à partir des pointes inférieures correspondantes pour des étapes de traitement qui sont requises afin d'obtenir la biomolécule désirée; et
(f) un moyen pour retirer les colonnes des têtes en vue de leur évacuation.

27. Assemblage de purification de biomolécule comprenant un récipient possédant une entrée de liquide et une unité de filtrage située de manière amovible sur l'entrée de liquide, dans lequel le récipient est en forme d'une colonne possédant une première section de conduite et une seconde section de conduite de diamètre réduit comparé à la première section, et l'unité de filtrage est constituée d'un manchon qui abrite le filtre et qui est situé de manière amovible sur l'extrémité du récipient loin de la première section de conduite.

28. Assemblage de purification de biomolécule comme revendiqué dans la revendication 27, dans lequel la surface du filtre adjacent à l'entrée du récipient possède une dépression et l'extrémité du récipient se situe dans la dépression.

29. Unité de filtrage pour un assemblage de purification de biomolécule selon la revendication 27 ou 28, ladite unité de filtrage comprenant
(a) un manchon allongé possédant une extrémité (l'extrémité d'entrée de la suspension) à travers laquelle une suspension à filtrer entre dans le manchon et une extrémité opposée destinée à être placée sur une colonne d'assemblage de purification de biomolécule, ledit manchon possédant une première partie de corps et une seconde partie de corps qui s'effile intérieurement à partir de la première partie de corps jusqu'à ladite extrémité d'entrée de la suspension, et
(b) un filtre possédant une partie de tête se situant dans, et occupant la section transversale de, la première partie de corps, et une partie de corps conique ou tronconique s'étendant dans ladite seconde partie de corps du manchon et s'effilant dans celui-ci suivant un angle plus grand que l'angle d'effilement interne du manchon, et l'unité de filtrage étant située de manière amovible dans ledit assemblage.

30. Assemblage de purification de biomolécule selon la revendication 27 ou 28 comprenant
(i) un récipient allongé en forme d'une colonne possédant une première et une seconde extrémité ouvertes avec une entrée de filtrat qui est prévue à la première extrémité de la colonne, et
(ii) l'unité de filtrage telle qu'elle est définie dans la revendication 29 située de manière amovible sur la première extrémité du récipient.

31. Assemblage comme revendiqué dans la revendication 30, dans lequel l'unité de filtrage possède une dépression supérieure et ladite entrée de filtrat du récipient allongé est située dans la région de ladite dépression.

32. Assemblage comme revendiqué dans la revendication 30 ou 31, dans lequel ledit récipient allongé est tel qu'il est défini dans la revendication 24.

33. Procédé comme revendiqué dans la revendication 1, dans lequel la suspension est obtenue grâce aux étapes consistant à:
(i) réaliser une re-suspension du granulé bactérien en solution;
(ii) ajouter de la solution de lyse à la re-suspension pour réaliser la libération des contenus cellulaires; et
(iii) ajouter de la solution neutralisante à la re-suspension lysée pour réaliser la formation d'une suspension comprenant une solution d'une biomolécule et du matériau insoluble pour utilisation dans le procédé de la revendication 1;
dans lequel la solution de lyse et/ou la solution neutralisante sont chacune ajoutées en parties aliquotes multiples qui sont éjectées vers le bas dans la re-suspension pour réaliser la suspension en l'absence de remuement ou d'agitation mécanique supplémentaire sans que l'appareil distributeur ne vienne en contact avec l'échantillon ou avec le récipient conteneur.

34. Procédé d'exploitation d'un appareil comme défini dans la revendication 26 utilisant les étapes consistant à lyser l'échantillon cellulaire et à obtenir la biomolécule à partir du lysat dans lequel les réactifs pour la lyse sont éjectés vers le bas à travers les têtes de support dans l'échantillon cellulaire à lyser (soit avant soit après le montage des colonnes sur les têtes).

35. Procédé comme revendiqué dans la revendication 34, dans lequel les réactifs de lyse comprennent une solution de lyse et une solution neutralisante séparée.

36. Procédé comme revendiqué dans une quelconque des revendications 33 à 35, dans lequel les échantillons cellulaires à lyser sont contenus à l'intérieur des puits individuels d'un plateau de puits pour microtitrage ou équivalent et les réactifs de lyse sont éjectés dans les puits individuels.

37. Procédé comme revendiqué dans la revendication 36, dans lequel l'appareil comprend une rangée simple de n têtes de support de colonne et les échantillons cellulaires à traiter sont agencés en un réseau (m x n) de puits pour microtitrage dans lequel les échantillons dans les n puits d'une quelconque des m rangées sont simultanément traités avec des réactifs de lyse par éjection vers le bas à partir des n têtes de support.

38. Procédé comme revendiqué dans la revendication 37, dans lequel les réactifs de lyse sont déchargés dans tous les puits du réseau (m x n) avant que les colonnes jetables ne soient montées sur les têtes pour les étapes ultérieures de la procédure d'extraction.

39. Procédé comme revendiqué dans une quelconque des revendications 33 à 38, dans lequel le temps de distribution du réactif de lyse dans un échantillon cellulaire est de 0,1 à 1 seconde.

40. Procédé comme revendiqué dans une quelconque des revendications 33 à 39, dans lequel la sortie de décharge des têtes de support de colonne est de 40 mm à 100 mm au-dessus de la surface de l'échantillon dans lequel les réactifs de lyse doivent être déchargés.

41. Procédé comme revendiqué dans la revendication 40, dans lequel ladite sortie de décharge est environ 70 mm au-dessus de ladite surface de l'échantillon.

42. Procédé comme revendiqué dans une quelconque des revendications 33 à 41, dans lequel la sortie de décharge de chacune des têtes de support possède un diamètre de 0,1 à 1 mm.

43. Procédé comme revendiqué dans la revendication 42, dans lequel ledit diamètre est d'environ 0,75 mm.
